# FASCICULE DE BREVET EUROPEEN

(11) **EP 4 126 217 B1**
(45) Date de publication et mention de la délivrance du brevet: **04.06.2025**
(21) Numéro de dépôt: 21722276.9
(22) Date de dépôt: 01.04.2021
(51) Int. Cl.: A61N 7/00, A61M 37/00

(54) **RUPTURE SÉLECTIVE ET RÉVERSIBLE DE LA BARRIÈRE HÉMATO-ENCÉPHALIQUE PAR ÉMISSION D'ULTRASONS**
SELEKTIVE UND UMKEHRBARE BRECHUNG DER BLUT-HIRN-SCHRANKE DURCH ULTRASCHALLEMISSION
SELECTIVE AND REVERSIBLE BREAKING OF THE BLOOD-BRAIN BARRIER BY ULTRASOUND EMISSION

(30) Priorité: 01.04.2020 FR 2003270
(43) Date de publication de la demande: 08.02.2023
(73) Titulaire: Brainwaves, 75019 Paris (FR)
(72) Inventeur: SADIK, Jean-Claude, 75005 Paris (FR); CRITON, Aline, 92100 Boulogne-Billancourt (FR)
(74) Mandataire: Icosa
(86) Numéro de dépôt international: PCT/FR2021/050575
(87) Numéro de publication internationale: WO 2021/198620

(56) Documents cités:
- EP-A1- 3 733 243
- WO-A1-2011/057028
- WO-A1-2019/131723
- US-B1- 7 896 821
- VALDEZ MICHAEL AARON: "Development, Characterization, and Implementation of a System for Focused Ultrasound-Mediated Blood-Brain Barrier Opening in Mice", 1 December 2017 (2017-12-01), United States -- Arizona, pages 1 - 188, XP093061843, ISBN: 978-0-355-52320-1, Retrieved from the Internet <URL:https://www.proquest.com/docview/1983517816> [retrieved on 20230706]
- ANTHONY NOVELL: "Droplet-assisted ultrasound for brain therapy using CMUT technology - DROPMUT", 16 October 2019 (2019-10-16), XP055746356, Retrieved from the Internet <URL:https://anr.fr/Project-ANR-19-CE19-0011> [retrieved on 20201103]
- DASGUPTA ANSHUMAN ET AL: "Ultrasound-mediated drug delivery to the brain: principles, progress and prospects", DRUG DISCOVERY TODAY: TECHNOLOGIES, ELSEVIER, AMSTERDAM, NL, vol. 20, 25 October 2016 (2016-10-25), pages 41 - 48, XP029847521, ISSN: 1740-6749, DOI: 10.1016/J.DDTEC.2016.07.007
- KANG-HO SONG ET AL: "State-of-the-art of microbubble-assisted blood-brain barrier disruption", THERANOSTICS, vol. 8, no. 16, 7 August 2018 (2018-08-07), AU, pages 4393 - 4408, XP055602152, ISSN: 1838-7640, DOI: 10.7150/thno.26869

## Description

### DOMAINE DE L'INVENTION

La présente invention concerne un dispositif configuré pour rompre sélectivement et réversiblement la barrière hémato-encéphalique d'un sujet en émettant des ultrasons.

Le présent document divulgue également une méthode pour améliorer la biodisponibilité dans les tissus cérébraux d'au moins un agent luttant contre les médiateurs de l'inflammation chez un sujet par rupture sélective et réversible de la barrière hémato-encéphalique d'un sujet. Une telle méthode ne fait pas partie de l'invention revendiquée.

Le présent document divulgue également l'utilisation du dispositif de la présente invention pour le traitement des maladies du système nerveux central. Une telle utilisation ne fait également cependant pas partie de l'invention revendiquée.

### ÉTAT DE LA TECHNIQUE

Les médiateurs de l'inflammation et particulièrement le Facteur de Nécrose Tumorale (TNF) est une composante essentielle du système immunitaire du cerveau et joue un rôle important dans la régulation de la transmission des impulsions nerveuses. En excès, il peut nuire à la bonne conduction de l'influx nerveux et provoquer une réaction inflammatoire.

Dans le système nerveux central (SNC), le facteur de nécrose tumorale alpha (TNF-alpha) dérive de la microglie activée et joue un rôle essentiel de médiateur inflammatoire. Il a notamment été montré par imagerie cérébrale que l'activation de microglie intracérébrale entrainait une neuro-inflammation intracérébrale après un accident vasculaire cérébral ou d'autres formes de lésions cérébrales, du fait d'un taux élevé de TNF-alpha relâché. Plusieurs analyses ont également montré que des patients atteint de la maladie d'Alzheimer avaient un taux élevé de TNF-alpha dans leur liquide céphalorachidien.

Généralement utilisés pour la prise en charge de maladies inflammatoires chroniques invalidantes, telles que la polyarthrite rhumatoïde, la spondylarthrite ankylosante, le psoriasis sévère et sa forme rhumatismale, les anti-TNF-alpha représentent une classe d'agents thérapeutiques intéressants dans le traitement des maladies du système nerveux central.

Cependant, dans le traitement des maladies du système nerveux central, la barrière hémato-encéphalique (BHE) représente un obstacle majeur. En effet, la barrière hémato-encéphalique est formée par des couches de cellules tapissant le système vasculaire cérébral et permettant ainsi une stabilité de l'environnement cérébral en prévenant l'entrée de nombreuses substances, généralement d'un poids moléculaire supérieur à 180 Da, telles que des toxines, virus, bactéries et agents thérapeutiques circulant dans le sang.

Ainsi, il est souvent observé dans le traitement des maladies du système nerveux central que seulement une très faible quantité de principe actif nécessaire pour traiter la pathologie parvient à traverser la barrière hémato-encéphalique. Les anti-TNF alpha connus étant des protéines de fusion ou anticorps monoclonaux, leur poids moléculaire dépasse généralement les 100 KDa et ils présentent donc des difficultés à passer la barrière hémato-encéphalique.

Il existe donc un besoin d'améliorer la biodisponibilité des anti-TNF-alpha afin de les utiliser dans le traitement des maladies du système nerveux central.

Dans ce but, des techniques d'ouverture sélective et réversible de la barrière hémato-encéphalique ont été développées afin de permettre à la quantité nécessaire d'agent thérapeutique d'atteindre les tissus cérébraux de manière sûre et contrôlée.

On connait par exemple le brevet US 7,896,821 portant sur une méthode et un appareil permettant une rupture réversible de la barrière hémato-encéphalique en utilisant des ultrasons focalisés de faible intensité. Anthony Novell et al. (« Droplet-assisted ultrasound for brain therapy using CMUT technology - DROPMUT », 2019) décrit une proposition de projet de recherche mené dans le cadre d'un financement ANR et ayant trait au traitement du syndrome de Rett par l'utilisation de la technologie ultrasons. On connait également la demande WO 2011/057028 portant sur un procédé et un dispositif permettant de moduler l'activité cérébrale chez l'homme en utilisant des ultrasons. Il n'a pas été démontré que le dispositif de la demande WO 2011/057028 permette d'agir sur les structures vasculaires du cerveau et ainsi de rompre de manière réversible la barrière hémato-encéphalique. L'article "State-of-the-art of microbubble-assisted blood-brain barrier disruption" (KANG-HO SONG ET AL, THERANOSTICS, vol. 8, no. 16, 7 août 2018, pages 4393-4408, XP055602152, ISSN: 1838-7640, DOI: 10.7150/thno.26869) résume l'état de la technique en ce qui concerne l'ouverture réversible de la barrière hémato-encéphalique par ultrasons assistée au moyen de microbulles.

Cependant, les transducteurs de l'art antérieur utilisés en thérapie ultrasonore utilisent généralement de longs trains d'ondes, proches du continu, ce qui génère de forts échauffements au sein des transducteurs. Il est donc généralement obligatoire d'utiliser un système de refroidissement par circulation d'eau, pour éviter les risques de détérioration du transducteur.

Aussi, aucun des dispositifs de l'art antérieur n'a été utilisé pour l'administration d'anti-TNF-alpha pour le traitement des maladies du système nerveux central.

La présente invention vise à répondre à ce besoin en proposant un dispositif configuré pour rompre sélectivement et réversiblement la barrière hémato-encéphalique d'un sujet en émettant des ultrasons. Le dispositif de la présente invention peut par exemple utiliser des transducteurs ultrasonores micro-usinés, présentant de faibles pertes mécaniques internes comparées aux transducteurs de l'art antérieur et donc une plus faible augmentation de la température.

### RÉSUMÉ

L'invention concerne donc un dispositif configuré pour rompre sélectivement et réversiblement la barrière hémato-encéphalique d'un sujet en émettant des ultrasons, comprenant : une structure configurée pour être placée sur au moins une portion de la tête d'un utilisateur ; et au moins deux transducteurs ultrasonores couplés à ladite structure et configurés pour émettre des ultrasons à intensité diagnostique. Ledit dispositif est adapté pour augmenter la biodisponibilité dans les tissus cérébraux d'au moins un agent luttant contre les médiateurs de l'inflammation. Dans un mode de réalisation préféré, l'agent luttant contre les médiateurs de l'inflammation est au moins un anti-inflammatoire. Dans un mode de réalisation encore plus préféré, l'agent luttant contre les médiateurs de l'inflammation est au moins un anti-TNF alpha.

Lesdits au moins deux transducteurs ultrasonores sont de type capacitif micro-usiné, piézoélectrique micro-usiné ou piézoélectrique.

Dans un mode de réalisation, ladite structure est constituée d'un casque, d'un bonnet, d'une cagoule ou d'un bandeau.

Dans un mode de réalisation, lesdits au moins deux transducteurs ultrasonores sont positionnés selon un plan frontal et/ou un plan sagittal.

Dans un mode de réalisation, lesdits au moins deux transducteurs ultrasonores sont mobiles selon un axe frontal et/ou un axe sagittal.

Dans un mode de réalisation, le dispositif comprend en outre un dispositif de contrôle couplé auxdits aux moins deux transducteurs ultrasonores et configuré pour contrôler la fréquence et la puissance des ultrasons émis par lesdits aux moins deux transducteurs ultrasonores.

Dans un mode de réalisation, lesdits au moins deux transducteurs ultrasonores reposent à la surface du cuir chevelu.

Dans un mode de réalisation, le dispositif comprend une pluralité de transducteurs ultrasonores formant un réseau de transducteurs à la surface du cuir chevelu.

Lesdits au moins deux transducteurs ultrasonores sont configurés pour délivrer une fréquence d'ultrasons comprise entre 1 et 4 MHz.

Lesdits au moins deux transducteurs ultrasonores sont configurés pour délivrer des ultrasons à une puissance comprise entre 400 et 800 mW/cm².

La fréquence et la puissance des ultrasons sont choisies de manière à ce qu'une énergie suffisante soit transférée à la boite crânienne du sujet afin de que les microbulles injectées entrent en oscillation au niveau d'au moins un site ciblé du cerveau permettant ainsi la rupture sélective de la barrière hémato-encéphalique par action mécanique.

### DÉFINITIONS

Dans la présente invention, les termes ci-dessous sont définis de la manière suivante :
- « **Administration** » ou une de ses variantes (par exemple « administrer »), signifie fournir l'agent actif ou le principe actif, seul ou dans une composition pharmaceutiquement acceptable au patient chez qui l'état, le symptôme ou la maladie doit être traité(e) ou prévenu(e). L'expression « **pharmaceutiquement acceptable** » désigne les ingrédients d'une composition pharmaceutique qui sont compatibles entre eux et qui ne sont pas nocifs pour le sujet auquel ils sont administrés.
- « **Anti-inflammatoire** » concerne une substance utilisée pour lutter contre l'inflammation, processus de défense de l'organisme face à une agression, caractérisé par des signes de chaleur, douleur, rougeur et tuméfaction. Dans un mode de réalisation, l'anti-inflammatoire est stéroïdien (type cortisone). Dans un mode de réalisation, l'anti-inflammatoire est non stéroïdien.
- « **Anti-TNF alpha** » concerne des médicaments issus de la biothérapie (aussi appelés biomédicaments ou biosimilaires par rapport à un anti-TNF alpha de référence). Dans un mode de réalisation, les anti-TNF alpha sont des anticorps monoclonaux complètement ou partiellement humanisés. Dans un mode de réalisation, les anti-TNF alpha sont des protéines chimériques se comportant comme des récepteurs solubles du TNF alpha. Les anti-TNF alpha diminuent le TNF alpha sérique, ce qui va permettre de contrôler l'inflammation régionale et donc l'évolution de ces pathologies. Dans un mode de réalisation, ils sont administrés de manière réitérée par perfusions intra-veineuses. Dans un mode de réalisation, ils sont administrés par injections sous-cutanées en association ou en monothérapie. Dans un mode de réalisation, les anti-TNF alpha sont choisis parmi l'Etanercept, l'Infliximab, l'Adalimumab, le Golimumab, le Certolizumab.
- « **Biodisponibilité** » concerne la proportion d'une substance qui atteint la circulation sanguine sous forme inchangée. Au sens de l'invention, concerne la proportion d'anti-inflammatoire, notamment d'anti-TNF alpha atteignant les tissus cérébraux sous forme inchangée.
- Les termes « **quantité thérapeutiquement efficace** » ou « **quantité efficace** » ou **« dose thérapeutiquement efficace** » se réfèrent à la quantité ou à la dose d'ingrédient actif qui vise, sans causer d'effets secondaires négatifs ou indésirables importants au sujet, à (1) réduire la gravité ou l'incidence de la maladie ; (2) ralentir ou arrêter la progression, l'aggravation ou la détérioration d'un ou de plusieurs symptômes de la maladie ciblée affectant le sujet ; (3) apporter des améliorations aux symptômes de la maladie ciblée affectant le sujet ; ou (4) guérir la maladie ciblée affectant le sujet.
- « **Environ** », placé devant un nombre, signifie plus ou moins 10% de la valeur nominale de ce nombre.
- « **Médiateur de l'inflammation** » concerne des médiateurs chimiques qui déclenchent et stimulent la réaction inflammatoire. Ce sont ces médiateurs qui sont responsables des manifestations caractéristiques de l'inflammation : augmentation de la perméabilité vasculaire, vasodilatation, fièvre et douleur. Des exemples non limitatifs de médiateurs de l'inflammation sont l'histamine, les prostaglandines, les cytokines pro-inflammatoires (TNF, IL1, et IL6).
- « **Plan sagittal** » concerne un plan allant de l'avant vers l'arrière et formant un angle droit avec le plan frontal. Il est parallèle au plan médian.
- « **Plan frontal** » concerne un plan perpendiculaire au plan sagittal et qui sépare le corps en une partie antérieure ou ventrale et une partie postérieure ou dorsale.
- « **Plan médian** » concerne un plan qui sépare la moitié gauche et la moitié droite du corps.
- « **Traiter** », « **soigner** » et « **traitement** », tels qu'ils sont utilisés dans la présente invention, font référence à un traitement thérapeutique, à l'exclusion des mesures prophylactiques ou préventives, dans lequel l'objectif est de ralentir (atténuer) une maladie donnée. Les personnes ayant besoin d'un traitement comprennent celles qui sont déjà atteintes de la maladie ainsi que celles que l'on soupçonne d'être atteintes de la maladie. Un sujet est « traité » avec succès pour une maladie donnée si, après avoir été traité avec la méthode pour améliorer la biodisponibilité des agents luttant contre les médiateurs de l'inflammation chez un sujet ou la méthode de traitement selon la présente invention, ledit sujet présente une réduction observable et/ou mesurable ou l'absence d'un ou plusieurs des éléments suivants : un ou plusieurs des symptômes associés à la maladie du système nerveux central ; une réduction de la morbidité et de la mortalité ; et/ou une amélioration de la qualité de vie. Les paramètres ci-dessus pour évaluer le succès du traitement et l'amélioration de la maladie du système nerveux central sont facilement mesurables par des procédures de routine familières à un médecin.
- « **Sujet** » désigne un mammifère, de préférence un être humain. Dans un mode de réalisation, un sujet peut être un « **patient** », c'est-à-dire un mammifère, plus préférablement un humain, qui attend de recevoir, ou reçoit des soins médicaux, ou a été/est/sera l'objet d'une procédure médicale, ou est surveillé pour le développement d'une maladie.
- Le terme « **mammifère** » désigne ici tout mammifère, y compris les humains, les animaux domestiques et de ferme, les animaux de zoo, les animaux de sport, etc., tels que les chiens, les chats, les bovins, les chevaux, les moutons, les porcs, chèvres, lapins, etc. De préférence, le mammifère est un primate, plus préférablement un humain. Le terme « **humain** » désigne un sujet des deux sexes et à n'importe quel stade de son développement (c'est-à-dire nouveau-né, nourrisson, mineur, adolescent, adulte). Dans un mode de réalisation, le sujet est un homme. Dans une autre forme de réalisation, le sujet est une femme. Dans un mode de réalisation, le sujet est un adulte. Dans une autre forme de réalisation, le sujet est un enfant.
- « **Transducteur ultrasonore** » concerne un système convertissant une énergie électrique en une énergie acoustique dans la gamme des ultrasons. Dans un mode de réalisation, le transducteur ultrasonore est un transducteur micro-usiné.
- « **Transducteur micro-usiné** » concerne un transducteur fabriqué selon les technologies des microsystèmes. Dans un mode de réalisation, le transducteur micro-usiné a une dimension comprise entre 10 et 100 microns. Dans un mode de réalisation, le transducteur ultrasonore est un transducteur capacitif micro-usiné (CMUT). Dans un mode de réalisation, le transducteur ultrasonore est un transducteur piézoélectrique micro-usiné (PMUT).
- « **Ultrason** » concerne une onde mécanique et élastique, qui se propage au travers de supports fluides, solides, gazeux ou liquides. Dans un mode de réalisation, la gamme de fréquences des ultrasons se situe entre 16 000 et 10 000 000 Hertz.
- « **Intensité diagnostique** » concerne une intensité des ultrasons inférieure à 800 mW/cm², plus précisément comprise entre 50 mW/ cm² et 800 mW/cm².

### BRÈVE DESCRIPTION DES FIGURES

**Figure 1** est une représentation schématique du dispositif d'émissions d'ultrasons selon un mode de réalisation de la présente invention.
**Figure 2** est une représentation schématique du dispositif d'émissions d'ultrasons selon un autre mode de réalisation de la présente invention.
**Figure 3** est une représentation schématique du dispositif d'émissions d'ultrasons selon un autre mode de réalisation de la présente invention.
**Figure 4** est un organigramme montrant les principales étapes de la méthode selon la présente invention.

### RÉFÉRENCES

1 - dispositif d'émission d'ultrasons
2 - structure
3 - transducteurs ultrasonores
4 - dispositif de contrôle

### DESCRIPTION DÉTAILLÉE

La description suivante sera mieux comprise à la lecture des dessins. Dans le but d'illustrer l'invention, le dispositif est représenté dans des modes de réalisation préférés. Il doit être compris, cependant, que la présente demande n'est pas limitée aux arrangements, structures, caractéristiques, modes de réalisation et apparence précis indiqués. Les dessins ne sont pas dessinés à l'échelle et ne sont pas destinés à limiter la portée des revendications aux modes de réalisation représentés dans ces dessins. Par conséquent, il doit être compris que lorsque des caractéristiques mentionnées dans les revendications sont suivies par des références, lesdites références sont incluses uniquement en vue d'améliorer la compréhension des revendications et ne limitent en aucun cas la portée de ces revendications.

### Dispositif

La présente invention concerne un dispositif d'émission d'ultrasons 1, configuré pour rompre sélectivement et réversiblement la barrière hémato-encéphalique d'un sujet, comprenant une structure 2 configurée pour être placée sur au moins une portion de la tête dudit sujet et au moins deux transducteurs ultrasonores 3 couplés à ladite structure 2, configurés pour émettre des ultrasons focalisés à intensité diagnostique. Ledit dispositif 1 est adapté pour augmenter la biodisponibilité dans les tissus cérébraux d'au moins un agent luttant contre les médiateurs de l'inflammation. Dans un mode de réalisation préféré, ledit dispositif 1 est adapté pour augmenter la biodisponibilité dans les tissus cérébraux d'au moins un anti-inflammatoire. Dans un mode de réalisation encore plus préféré, ledit dispositif 1 est adapté pour augmenter la biodisponibilité dans les tissus cérébraux d'au moins un anti-TNF alpha.

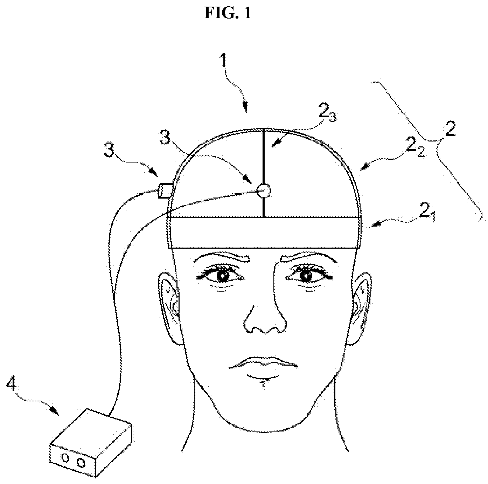

La figure 1 montre un mode de réalisation selon l'invention dans lequel le dispositif 1 comprend une structure 2 constituée d'un bandeau adapté pour entourer la tête d'un sujet, de préférence le bandeau est constitué d'une bande circulaire 2₁ configurée pour encercler la tête du sujet au niveau des tempes, d'une première bande supérieure 2₂ reliée à la bande circulaire selon un plan frontal et d'une deuxième bande supérieure 2₃ reliée à la bande circulaire selon un plan sagittal. Les deux bandes supérieures 2₂ et 2₃ sont configurées pour reposer sur le haut de la tête du sujet. Dans ce mode de réalisation, deux transducteurs ultrasonores 3 sont couplés au bandeau 2. Un premier transducteur temporal est couplé à la première bande supérieure frontale 2₂ et le deuxième transducteur frontal est couplé à la deuxième bande supérieure sagittale 2₃. Dans ce mode de réalisation, les deux transducteurs 3 sont mobiles et sont positionnés de façon à balayer la totalité de la surface du cerveau du sujet. Le transducteur temporal est mobile selon au choix un axe frontal ou transversal et le transducteur frontal est mobile selon un axe sagittal. Comme montré sur la figure 1, les deux transducteurs 3 reposent à la surface du cuir chevelu. Comme montré sur la figure 1, les transducteurs ultrasonores 3 sont couplés à un dispositif de contrôle 4 configuré pour contrôler la fréquence et la puissance des ultrasons émis par lesdits transducteurs ultrasonores 3. Dans ce mode de réalisation, le dispositif de contrôle 4 est couplé aux transducteurs ultrasonores 3 par un câble électrique.

Au moins deux transducteurs ultrasonores sont couplés à la structure 2. Dans un mode de réalisation, au moins un transducteur ultrasonore est couplé à la structure 2 selon un plan frontal. Dans un mode de réalisation, au moins un transducteur ultrasonore est couplé à la structure 2 selon un plan sagittal. Dans un mode de réalisation, les au moins deux transducteurs ultrasonores sont couplés à la structure 2 selon un plan frontal. Dans un mode de réalisation, les au moins deux transducteurs ultrasonores sont couplés à la structure 2 selon un plan sagittal.

Selon un mode de réalisation, les au moins deux transducteurs ultrasonores sont mobiles. Dans un mode de réalisation, les au moins deux transducteurs ultrasonores sont mobiles selon un axe frontal. Dans un mode de réalisation, les au moins deux transducteurs ultrasonores sont mobiles selon un axe sagittal. Dans ces modes de réalisation, un balayage de part et d'autre du plan médian est possible.

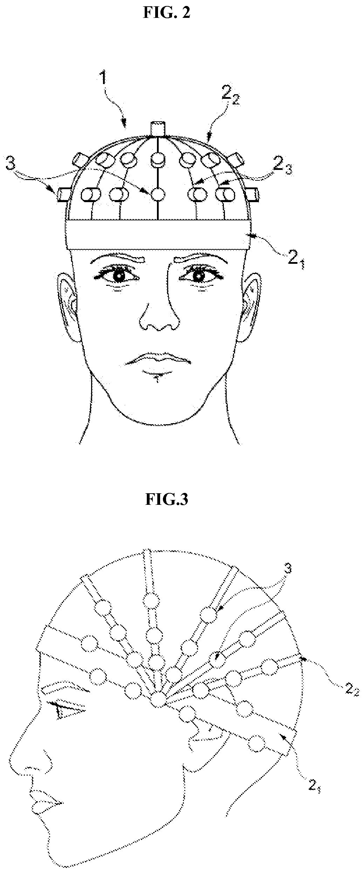

La figure 2 montre un autre mode de réalisation selon l'invention dans lequel le dispositif 1 décrit à la figure 1 est apte à recevoir une pluralité de transducteurs ultrasonores 3. Selon un mode de réalisation, pluralité de transducteurs ultrasonores 3 signifie de trois à cinq cents transducteurs ultrasonores. Selon un mode de réalisation, pluralité de transducteurs ultrasonores 3 signifie au moins trois transducteurs ultrasonores. Selon un mode de réalisation, pluralité de transducteurs ultrasonores 3 signifie au moins quatre transducteurs ultrasonores. Selon un mode de réalisation, pluralité de transducteurs ultrasonores 3 signifie au moins cinq transducteurs ultrasonores. Selon un mode de réalisation, pluralité de transducteurs ultrasonores 3 signifie au moins dix transducteurs ultrasonores. Selon un mode de réalisation, pluralité de transducteurs ultrasonores 3 signifie au moins quinze transducteurs ultrasonores. Selon un mode de réalisation, pluralité de transducteurs ultrasonores 3 signifie au moins vingt transducteurs ultrasonores. Selon un mode de réalisation, pluralité de transducteurs ultrasonores 3 signifie au moins cinquante transducteurs ultrasonores. Selon un mode de réalisation, pluralité de transducteurs ultrasonores 3 signifie au moins cent transducteurs ultrasonores. Selon un mode de réalisation, pluralité de transducteurs ultrasonores 3 signifie au moins deux cents transducteurs ultrasonores. Selon un mode de réalisation, pluralité de transducteurs ultrasonores 3 signifie au moins trois cents transducteurs ultrasonores. Selon un mode de réalisation, pluralité de transducteurs ultrasonores 3 signifie au moins quatre cents transducteurs ultrasonores. Dans ce mode de réalisation, la pluralité de transducteurs ultrasonores 3 forme un réseau de transducteurs à la surface du cuir chevelu. Comme montré sur la figure 2, les transducteurs ultrasonores sont couplés à une pluralité de bandes selon un plan sagittal.

Dans un mode de réalisation représenté à la figure 3, les transducteurs d'une pluralité de transducteurs ultrasonores 3 sont couplés à la structure 2 au niveau de la zone temporale. Dans ce mode de réalisation, l'émission d'ultrasons est concentrée sur la zone temporale d'un sujet.

Selon un mode de réalisation, la structure 2 est configurée pour recouvrir entièrement la tête d'un sujet. Dans un mode de réalisation, la structure 2 est configurée pour recouvrir partiellement la tête d'un sujet. Dans un mode de réalisation, la structure 2 est configurée pour entourer la tête d'un sujet, de préférence au niveau des tempes dudit sujet.

Selon un mode de réalisation, la structure 2 est constituée d'un casque. Dans un mode de réalisation, la structure 2 est constituée d'un bonnet. Dans un mode de réalisation, la structure 2 est constituée d'une cagoule. Selon un mode de réalisation, la structure 2 est constituée d'un bandeau.

Les au moins deux transducteurs ultrasonores sont choisis parmi les transducteurs ultrasonores capacitifs micro-usinés (CMUT), les transducteurs ultrasonores piézoélectriques micro-usinés (PMUT) ou les transducteurs piézoélectriques. Dans un mode de réalisation préféré, les au moins deux transducteurs ultrasonores sont de type capacitifs micro-usinés (CMUT) ou ultrasonores piézoélectriques micro-usinés (PMUT).

Dans un mode de réalisation préféré, les au moins deux transducteurs ultrasonores sont de type capacitif micro-usiné (CMUT).

Selon un mode de réalisation, les au moins deux transducteurs ultrasonores sont constitués d'au moins une barrette comprenant au moins deux éléments. Dans un mode de réalisation, les au moins deux transducteurs ultrasonores sont des éléments isolés.

Selon un mode de réalisation, le dispositif de contrôle 4 est externe au dispositif 1. Dans un mode de réalisation, le dispositif de contrôle 4 est couplé auxdits au moins deux transducteurs 3 par un câble électrique. Dans un mode de réalisation, le dispositif de contrôle 4 est intégré auxdits au moins deux transducteurs 3.

Dans un mode de réalisation, le dispositif de contrôle 4 est configuré pour générer un signal électrique permettant d'activer les au moins deux transducteurs ultrasonores 3.

Dans un mode de réalisation, le dispositif de contrôle 4 est configuré pour générer des signaux séparément afin d'activer indépendamment les uns des autres chacun desdits au moins deux transducteurs ultrasonores 3. Dans un mode de réalisation, le dispositif de contrôle 4 est configuré pour générer un signal unique permettant l'activation simultanée de tous les transducteurs ultrasonores 3.

Dans un mode de réalisation, le dispositif de contrôle 4 est configuré pour contrôler différents paramètres desdits au moins deux transducteurs ultrasonores 3. Dans un mode de réalisation, le dispositif de contrôle 4 est configuré pour contrôler la fréquence des ultrasons émis par lesdits au moins deux transducteurs ultrasonores 3. Dans un mode de réalisation, le dispositif de contrôle 4 est configuré pour contrôler la puissance des ultrasons émis par lesdits au moins deux transducteurs ultrasonores 3. Dans un mode de réalisation, le dispositif de contrôle 4 est configuré pour contrôler l'amplitude des ultrasons émis par lesdits au moins deux transducteurs ultrasonores 3.

Lesdits au moins deux transducteurs ultrasonores sont configurés pour délivrer une fréquence d'ultrasons comprise entre 1 et 4 MHz. Dans un mode de réalisation préférée, lesdits au moins deux transducteurs ultrasonores sont configurés pour délivrer une fréquence d'ultrasons d'environ 1,5 MHz.

Lesdits au moins deux transducteurs ultrasonores sont configurés pour délivrer des ultrasons à une puissance comprise entre 400 et 800 mW/cm².

Dans un mode de réalisation, lesdits au moins deux transducteurs ultrasonores sont configurés pour délivrer des ultrasons à une puissance comprise entre 400 et 600 mW/cm². Dans un mode de réalisation, lesdits au moins deux transducteurs ultrasonores sont configurés pour délivrer des ultrasons à une puissance comprise entre 600 et 800 mW/cm². Dans un mode de réalisation préféré, lesdits au moins deux transducteurs ultrasonores sont configurés pour délivrer des ultrasons à une puissance de 800 mW/cm².

Dans un mode de réalisation, lesdits au moins deux transducteurs ultrasonores 3 reposent à la surface du cuir chevelu d'un sujet. Dans ce mode de réalisation, une pression uniforme est exercée sur la tête du sujet permettant ainsi de minimiser la perte d'énergie et l'effet d'échauffement dû aux ultrasons émis.

### Méthode

Le présent document concerne également une méthode pour améliorer la biodisponibilité des agents luttant contre les médiateurs de l'inflammation chez un sujet. Une telle méthode ne fait pas partie de l'invention. Dans ce but, la méthode est configurée pour émettre des ultrasons focalisés sur au moins un site spécifique du cerveau d'un sujet afin de rompre sélectivement et réversiblement la barrière hémato-encéphalique.

Ainsi, cette méthode pour améliorer la biodisponibilité dans au moins une région du tissu cérébral d'au moins un agent luttant contre les médiateurs de l'inflammation chez un sujet qui en a besoin comprend les étapes suivantes : administrer audit sujet une dose thérapeutiquement efficace d'au moins un agent luttant contre les médiateurs de l'inflammation, injecter des microbulles de gaz audit sujet, appliquer le dispositif de la présente invention sur au moins une portion de la tête dudit sujet, et émettre des ultrasons focalisés sur au moins un site du cerveau dudit sujet avec ledit dispositif, de manière préférentielle sur tout le tissu cérébral.

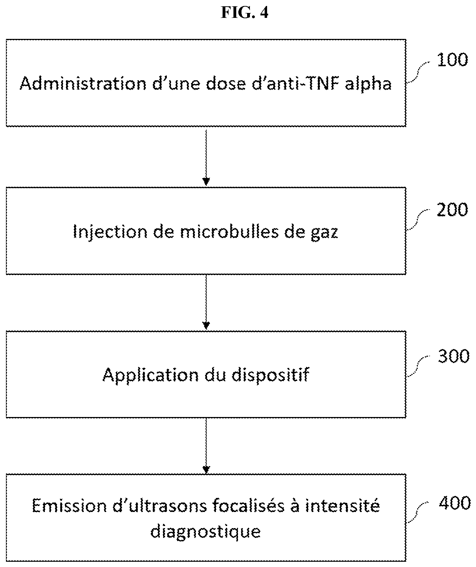

Comme montré sur la figure 4, la méthode comprend quatre étapes principales. Dans une première étape 100, une dose d'agents luttant contre les médiateurs de l'inflammation est administrée à un sujet. La seconde étape 200 consiste à injecter des microbulles de gaz audit sujet. Dans une troisième étape 300, le dispositif de la présente invention est appliqué sur au moins une portion de la tête dudit sujet, de manière préférentielle à la surface du crâne dudit sujet. Enfin, la quatrième étape 400 consiste à émettre des ultrasons focalisés sur au moins un site du cerveau.

Selon un mode de réalisation, l'étape d'administration d'une dose d'anti- TNF alpha 100 est réalisée avant l'injection de microbulles de gaz 200, qui est elle-même réalisée avant ou concomitamment à l'émission d'ultrasons focalisés à intensité diagnostique 400 sur au moins un site du cerveau dudit sujet. L'étape 300 pendant laquelle le dispositif de la présente invention est appliqué sur au moins une portion de la tête dudit sujet peut être réalisée avant l'administration d'une dose d'anti-TNF alpha 100, entre administration d'une dose d'anti-TNF alpha 100 et l'injection de microbulles de gaz 200, ou entre injection de microbulles de gaz 200 et l'émission d'ultrasons focalisés à intensité diagnostique 400 sur au moins un site du cerveau dudit sujet.

Dans un mode de réalisation, une dose d'au moins un agent luttant contre les médiateurs de l'inflammation est administrée au sujet soumis aux ultrasons. Dans un mode de réalisation, une dose d'au moins un agent luttant contre les médiateurs de l'inflammation est administrée au sujet au moins une fois par semaine. Dans un mode de réalisation, une dose d'au moins un agent luttant contre les médiateurs de l'inflammation est administrée au sujet au moins deux fois par semaine. Dans un mode de réalisation, une dose d'au moins un agent luttant contre les médiateurs de l'inflammation est administrée au sujet au moins trois jours par semaine. Dans un mode de réalisation, une dose d'au moins un agent luttant contre les médiateurs de l'inflammation est administrée au sujet au moins quatre jours par semaine. Dans un mode de réalisation, une dose d'au moins un agent luttant contre les médiateurs de l'inflammation est administrée au sujet au moins cinq jours par semaine. Dans un mode de réalisation, une dose d'au moins un agent luttant contre les médiateurs de l'inflammation est administrée au sujet au moins six jours par semaine. Dans un mode de réalisation, une dose d'au moins un agent luttant contre les médiateurs de l'inflammation est administrée au sujet sept jours par semaine.

Dans un mode de réalisation, une dose d'au moins un agent luttant contre les médiateurs de l'inflammation est administrée au sujet pendant au moins 24 semaines. Dans un mode de réalisation, une dose d'au moins un agent luttant contre les médiateurs de l'inflammation est administrée au sujet pendant au moins 2 semaines. Dans un mode de réalisation, une dose d'au moins un agent luttant contre les médiateurs de l'inflammation est administrée au sujet pendant au moins 4 semaines. Dans un mode de réalisation, une dose d'au moins un agent luttant contre les médiateurs de l'inflammation est administrée au sujet pendant au moins 8 semaines. Dans un mode de réalisation, une dose d'au moins un agent luttant contre les médiateurs de l'inflammation est administrée au sujet pendant au moins 12 semaines.

Dans un mode de réalisation, une dose d'au moins un agent luttant contre les médiateurs de l'inflammation est administrée au sujet au moins une fois par semaine et pendant au moins 24 semaines.

En pratique, la dose thérapeutiquement efficace d'agents luttant contre les médiateurs de l'inflammation à administrer dépend d'un ou de plusieurs paramètres, dont notamment, le matériel utilisé pour l'administration, l'âge, le sexe, la taille, le poids, la condition physique et le degré de sévérité du trouble à traiter. L'homme du métier connait la dose thérapeutiquement efficace d'agents anti-TNF alpha à administrer pour lutter contre les médiateurs de l'inflammation.

Dans un mode de réalisation, une dose d'au moins 25 mg d'au moins un agent luttant contre les médiateurs de l'inflammation est administrée au sujet. Dans un mode de réalisation, une dose de 25 mg d'au moins un agent luttant contre les médiateurs de l'inflammation est administrée au sujet. Dans un mode de réalisation, une dose de 50 mg d'au moins un agent luttant contre les médiateurs de l'inflammation est administrée au sujet. Dans un mode de réalisation, la dose d'au moins un agent luttant contre les médiateurs de l'inflammation est administrée par injection sous cutanée.

Selon un mode de réalisation préféré, ledit au moins un agent luttant contre les médiateurs de l'inflammation est un anti-inflammatoire. Selon un mode de réalisation plus préféré, ledit au moins un agent luttant contre les médiateurs de l'inflammation est un anti-TNF-alpha.

Dans un mode de réalisation, l'anti-TNF alpha est choisi parmi l'Etanercept, l'Infliximab, l'Adalimumab, le Golimumab, le Certolizumab. Dans un mode de réalisation préféré, l'anti-TNF alpha administré est l'Etanercept.

Selon un mode de réalisation, les microbulles de gaz sont injectées audit sujet par voie intraveineuse. Les microbulles de gaz injectées audit sujet sont des microbulles d'hexafluorure de soufre ou de perfluorocarbone tel que le perfluorohexane, le perfluoropropane, perfluoropentane, étant entendu que ces microbulles peuvent être stabilisées ou encapsulées.

Dans ce mode de réalisation, un agent de contraste ultrasonore est de préférence injecté audit sujet afin de générer les microbulles de gaz dans le sang. Dans un mode de réalisation, une solution d'hexafluorure de soufre est injectée audit sujet. Dans un mode de réalisation, une solution comprenant du iodipamide di-ester ou du diatrizoate est injectée audit sujet. Dans un mode de réalisation, une solution comprenant du bromure de perfluorooctyle ou du perfluorocarbone tel que le perfluorohexane, l'octafluoropropane, dodecafluoropentane, perfluoropentane est injectée audit sujet. Dans un mode de réalisation, une solution comprenant de l'Echovist^{®}, du Perflubron^{™}, de l'IDE, Sonavist^{®}, Levovist^{®}, Albunex^{®}, EchoGen^{®}, Optison^{®}, SonoVue^{®}, Definity^{®}, Imagent^{™}, Imavist^{®}, Sonazoïde, Quantison^{™}, Myomap^{™} ou SonoGen^{®} est injectée audit sujet.

Selon un mode de réalisation, les microbulles ont un diamètre moyen d'environ 2,5 µm.

De manière générale, les microbulles ont un diamètre compris entre 2 µm et 11 µm.

Dans un mode de réalisation, les microbulles ont un diamètre compris entre 2 µm et 6 µm.

Selon un mode de réalisation, le dispositif de la présente invention est appliqué de façon à recouvrir entièrement la tête d'un sujet. Dans un mode de réalisation, le dispositif de la présente invention est appliqué de façon à recouvrir partiellement la tête d'un sujet. Dans un mode de réalisation, le dispositif de la présente invention est appliqué de façon à recouvrir ou entourer la tête d'un sujet, de préférence au niveau des tempes dudit sujet. Dans un mode de réalisation, le dispositif de la présente invention est appliqué de façon à ce que les au moins deux transducteurs ultrasonores reposent à la surface du cuir chevelu.

Dans un mode de réalisation, ledit sujet est soumis à des ultrasons focalisés sur au moins un site spécifique du cerveau dudit sujet. Dans un mode de réalisation, ledit sujet est soumis à des ultrasons pendant une période comprise entre 5 min et 1 heure. Dans un mode de réalisation, ledit sujet est soumis à des ultrasons pendant une période comprise entre 5 min et 30 min. Dans un mode de réalisation, ledit sujet est soumis à des ultrasons pendant une période comprise entre 30 min et 1 heure. Dans un mode de réalisation, ledit sujet est soumis à des ultrasons pendant une période d'au moins 30 minutes. Dans un mode de réalisation préféré, ledit sujet est soumis à des ultrasons pendant une période de 30 minutes.

Dans un mode de réalisation, ledit sujet est soumis à des ultrasons au moins une fois par jour. Dans un mode de réalisation, ledit sujet est soumis à des ultrasons au moins deux fois par jour. Dans un mode de réalisation, ledit sujet est soumis à des ultrasons deux fois par jour.

Dans un mode de réalisation, ledit sujet est soumis à des ultrasons au moins un jour par semaine. Dans un mode de réalisation, ledit sujet est soumis à des ultrasons au moins deux jours par semaine. Dans un mode de réalisation, ledit sujet est soumis à des ultrasons au moins trois jours par semaine. Dans un mode de réalisation, ledit sujet est soumis à des ultrasons au moins quatre jours par semaine. Dans un mode de réalisation, ledit sujet est soumis à des ultrasons au moins cinq jours par semaine. Dans un mode de réalisation, ledit sujet est soumis à des ultrasons au moins six jours par semaine. Dans un mode de réalisation, ledit sujet est soumis à des ultrasons sept jours par semaine.

Dans un mode de réalisation, le sujet est soumis à des ultrasons pendant au moins 2 semaines. Dans un mode de réalisation, le sujet est soumis à des ultrasons pendant au moins 4 semaines. Dans un mode de réalisation, le sujet est soumis à des ultrasons pendant au moins 8 semaines. Dans un mode de réalisation, le sujet est soumis à des ultrasons pendant au moins 12 semaines. Dans un mode de réalisation, le sujet est soumis à des ultrasons pendant au moins 24 semaines. Dans un mode de réalisation, le sujet est soumis à des ultrasons pendant 24 semaines.

Selon un mode de réalisation préféré, le sujet est soumis à des ultrasons pendant 30 minutes, deux fois par jour, au moins cinq jours par semaine et pendant au moins 24 semaines.

Le sujet est soumis à des ultrasons à une fréquence comprise entre 1 et 4 MHz. Dans un mode de réalisation, le sujet est soumis à des ultrasons à une fréquence de 1,5 MHz.

Le sujet est soumis à des ultrasons à une puissance comprise entre 400 et 800 mW/cm². Dans un mode de réalisation, le sujet est soumis à des ultrasons à une puissance comprise entre 400 et 600 mW/cm². Dans un mode de réalisation, le sujet est soumis à des ultrasons à une puissance de 800 mW/cm². Selon un mode de réalisation, les étapes 200 à 400 sont répétées autant de fois que nécessaire.

Comme décrit plus haut, le dispositif de la présente invention permet une rupture sélective et réversible de la barrière hémato-encéphalique par émission d'ultrasons focalisés sur au moins un site spécifique du cerveau dudit sujet. La rupture localisée de la barrière hémato-encéphalique permet à la dose de modulateur de l'inflammation, c'est-à-dire agent luttant contre les médiateurs de l'inflammation, administrée circulant dans le sang de pénétrer plus efficacement à travers la région ouverte de la barrière hémato-encéphalique, délivrant ainsi la dose efficace nécessaire pour le traitement de la maladie ciblée.

Selon un mode de réalisation, la méthode pour améliorer la biodisponibilité des agents luttant contre les médiateurs de l'inflammation selon l'invention est utilisée pour le traitement des maladies du système nerveux central.

Le présent document concerne également une méthode de traitement des maladies du système nerveux central mettant en œuvre la méthode de la présente invention permettant d'améliorer la biodisponibilité des agents luttant contre les médiateurs de l'inflammation chez un sujet qui en a besoin. Une telle méthode ne fait pas partie de l'invention revendiquée.

Le présent document concerne également une méthode de traitement des maladies du système nerveux central mettant en œuvre le dispositif de la présente invention.

Une telle méthode ne fait également pas partie de l'invention revendiquée.

Selon un mode de réalisation, le dispositif de la présente invention est utilisé pour le traitement des maladies du système nerveux central.

Dans un mode de réalisation, la maladie du système nerveux central est choisie parmi la maladie d'Alzheimer, la maladie de Parkinson, l'épilepsie, les maladies cérébro-vasculaires, y compris l'accident vasculaire cérébral, la migraine, la sclérose en plaques, les infections du système nerveux, les tumeurs du cerveau, les troubles traumatiques du système nerveux tels que les traumatismes crâniens, la dépression et les troubles neurologiques liés à la malnutrition.

Bien que divers modes de réalisation aient été décrits et illustrés, la description détaillée ne doit pas être considérée comme étant limitée à ces derniers et l'invention est définie par les revendications jointes.

## Revendications

1. Dispositif (1) configuré pour rompre sélectivement et réversiblement la barrière hémato-encéphalique d'un sujet en émettant des ultrasons, comprenant :
- une structure (2) configurée pour être placée sur au moins une portion de la tête d'un utilisateur ;
- au moins deux transducteurs ultrasonores (3) couplés à ladite structure et configurés pour émettre des ultrasons à intensité diagnostique ;
lesdits au moins deux transducteurs ultrasonores (3) étant de type capacitif micro-usiné, piézoélectrique micro-usiné ou piézoélectrique et étant adaptés pour augmenter la biodisponibilité dans les tissus cérébraux d'au moins un agent luttant contre les médiateurs de l'inflammation, de préférence d'au moins un anti-inflammatoire, encore plus préférentiellement d'au moins un anti-TNF alpha ;
lesdits au moins deux transducteurs ultrasonores (3) sont configurés en ce que des microbulles préalablement injectées audit sujet entrent en oscillation et augmentent la porosité vasculaire au niveau d'au moins un site ciblé du cerveau dudit sujet, les microbulles ayant un diamètre compris entre 2 et 11 µm et étant des microbulles d'hexafluorure de soufre ou perfluorocarbone,
**caractérisé en ce que** lesdits au moins deux transducteurs ultrasonores (3) sont configurés pour délivrer une fréquence d'ultrasons comprise entre 1 et 4 MHz, et une puissance d'ultrasons émise comprise entre 400 et 800 mW/cm².

2. Dispositif selon la revendication **1, caractérisé en ce que** le perfluorocarbone est sélectionné parmi le perfluorohexane, le perfluoropropane, ou le perfluoropentane.

3. Dispositif selon l'une quelconque des revendications **1** ou **2, caractérisé en ce que** ladite structure (2) est constituée d'un casque, d'un bonnet, d'une cagoule ou d'un bandeau.

4. Dispositif selon l'une quelconque des revendications **1** à **3, caractérisé en ce que** lesdits au moins deux transducteurs ultrasonores (3) sont positionnés selon un plan frontal et/ou un plan sagittal.

5. Dispositif selon l'une quelconque des revendications **1** à **4, caractérisé en ce que** lesdits au moins deux transducteurs ultrasonores (3) sont mobiles selon un axe frontal et/ou un axe sagittal.

6. Dispositif selon l'une quelconque des revendications **1** à **5, caractérisé en ce qu'**il comprend en outre un dispositif de contrôle (4) couplé auxdits aux moins deux transducteurs ultrasonores (3) et configuré pour contrôler la fréquence et la puissance des ultrasons émis par lesdits aux moins deux transducteurs ultrasonores.

7. Dispositif selon l'une quelconque des revendications **1** à **6, caractérisé en ce que** lesdits au moins deux transducteurs ultrasonores (3) sont configurés pour reposer à la surface du cuir chevelu.

8. Dispositif selon l'une quelconque des revendications **1** à **7, caractérisé en ce qu'**il comprend une pluralité de transducteurs ultrasonores (3) configurés pour former un réseau de transducteurs à la surface du cuir chevelu.

## Patentansprüche

1. Vorrichtung (1), die so eingerichtet ist, dass sie die Blut-Hirn-Schranke eines Patienten durch Abgabe von Ultraschall selektiv und reversibel durchbricht, umfassend:
- eine Struktur (2), die so eingerichtet ist, dass sie auf mindestens einem Abschnitt des Kopfes eines Benutzers platziert wird;
- mindestens zwei Ultraschallwandler (3), die an die Struktur gekoppelt und zum Senden von Ultraschall mit diagnostischer Intensität eingerichtet sind;
die mindestens zwei Ultraschallwandler (3) vom mikrobearbeiteten kapazitiven, mikrobearbeiteten piezoelektrischen oder piezoelektrischen Typ sind und geeignet sind, um die Bioverfügbarkeit in Gehirngewebe von mindestens einem Mittel zur Bekämpfung von Entzündungsmediatoren, vorzugsweise von mindestens einem entzündungshemmenden Mittel, noch bevorzugter von mindestens einem TNF-Alpha-Antikörper, zu erhöhen;
die mindestens zwei Ultraschallwandler (3) so eingerichtet sind, dass zuvor in den Probanden injizierte Mikrobläschen in Schwingung geraten und die Gefäßporosität an mindestens einer Zielstelle des Gehirns des Probanden erhöhen, wobei die Mikrobläschen einen Durchmesser zwischen 2 und 11 µm haben und Schwefelhexafluorid- oder Perfluorkohlenstoff-Mikrobläschen sind, **dadurch gekennzeichnet, dass** die mindestens zwei Ultraschallwandler (3) so konfiguriert sind, dass sie eine Ultraschallfrequenz von 1 bis 4 MHz und eine Ultraschallleistung 2 von 400 bis 800 mW/cm² abgeben.

2. Vorrichtung nach Anspruch **1, dadurch gekennzeichnet, dass** das Perfluorkohlenstoff unter Perfluorhexan, Perfluorpropan oder Perfluorpentan ausgewählt ist.

3. Vorrichtung nach einem der Ansprüche **1** oder **2, dadurch gekennzeichnet, dass** die Struktur (2) aus einem Helm, einer Mütze, einer Haube oder einem Stirnband besteht.

4. Vorrichtung nach einem der Ansprüche **1** bis **3, dadurch gekennzeichnet, dass** die mindestens zwei Ultraschallwandler (3) in einer Stirn- und/oder Sagittalebene positioniert sind.

5. Vorrichtung nach einem der Ansprüche **1** bis **4, dadurch gekennzeichnet, dass** die mindestens zwei Ultraschallwandler (3) entlang einer Stirnachse und/oder einer Sagittalachse beweglich sind.

6. Vorrichtung nach einem der Ansprüche **1** bis **5, dadurch gekennzeichnet, dass** sie ferner eine Steuervorrichtung (4) umfasst, die mit den mindestens zwei Ultraschallwandlern (3) gekoppelt und zur Steuerung der Frequenz und Leistung des von den mindestens zwei Ultraschallwandlern ausgestrahlten Ultraschalls eingerichtet ist.

7. Vorrichtung nach einem der Ansprüche **1** bis **6, dadurch gekennzeichnet, dass** die mindestens zwei Ultraschallwandler (3) so eingerichtet sind, dass sie auf der Kopfhautoberfläche aufliegen.

8. Vorrichtung nach einem der Ansprüche **1** bis **7, dadurch gekennzeichnet, dass** sie eine Vielzahl von Ultraschallwandlern (3) umfasst, die so eingerichtet sind, dass sie ein Netzwerk von Wandlern an der Kopfhautoberfläche bilden.

## Claims

1. A device (1) configured to selectively and reversibly break a subject's blood-brain barrier by emitting ultrasound, comprising:
- a structure (2) configured to be placed on at least a portion of a user's head;
- at least two ultrasound transducers (3) coupled to said structure and configured to emit ultrasound at diagnostic intensity;
said at least two ultrasonic transducers (3) being of the capacitive micromachined, piezoelectric micromachined or piezoelectric type and being adapted to increase the bioavailability in brain tissue of at least one agent combating inflammatory mediators, preferably at least one anti-inflammatory agent, even more preferably at least one anti-TNF alpha agent;
said at least two ultrasound transducers (3) are configured so that microbubbles previously injected into said subject enter into oscillation and increase vascular porosity at at least one targeted site in said subject's brain, the microbubbles having a diameter of between 2 and 11 µm and being microbubbles of sulfur hexafluoride or perfluorocarbon
**characterized in that** said at least two ultrasound transducers (3) are configured to deliver an ultrasound frequency of between 1 and 4 MHz, and an emitted ultrasound power of between 400 and 800 mW/cm².

2. Device according to claim 1, **characterized in that** perfluorocarbon is selected among perfluorohexane, perfluoropropane, or perfluoropentane.

3. Device according to claim 1 or claim 2, **characterized in that** said structure (2) consists of a helmet, cap, hood or headband.

4. Device according to any one of claims 1 to 3, **characterized in that** said at least two ultrasonic transducers (3) are positioned in a frontal plane and/or a sagittal plane.

5. Device according to any one of claims 1 to 4, **characterized in that** said at least two ultrasonic transducers (3) are movable along a frontal axis and/or a sagittal axis.

6. Device according to any one of claims 1 to 5, **characterized in that** it further comprises a control device (4) coupled to said at least two ultrasonic transducers (3) and configured to control the frequency and power of the ultrasound emitted by said at least two ultrasonic transducers.

7. Device according to any one of claims 1 to 6, **characterized in that** said at least two ultrasonic transducers (3) are configured to rest on the surface of the scalp.

8. Device according to any one of claims 1 to 7, **characterized in that** it comprises a plurality of ultrasonic transducers (3) configured to form a transducer array on the surface of the scalp.
